# EUROPEAN PATENT APPLICATION

(11) **EP 1 959 007 A1**
(43) Date of publication of application: **20.08.2008**
(21) Application number: 07450029.9
(22) Date of filing: 16.02.2007
(51) Int. Cl.: C12N 5/06, C07H 21/00

(54) **Method for providing mature dendritic cells**

(71) Applicant: Romani, Nikolaus, 6020 Innsbruck (AT)
(72) Inventor: Romani, Nikolaus, 6020 Innsbruck (AT)
(74) Representative: Sonn & Partner Patentanwälte

(57) **Abstract**

The invention describes a method for providing mature dendritic cells (DCs) which is characterised by the following steps:
- providing a monocyte preparation,
- treating the monocyte preparation with a culturing mixture comprising interleukin 4 (IL-4) to obtain a cultured preparation comprising immature DCs,
- treating the cultured preparation with a maturing mixture comprising a ligand for the toll-like receptor 3 (TLR-3 ligand) to obtain a preparation comprising mature dendritic cells (DCs).

## Description

The present invention relates to the providing of dendritic cells (DCs).

Dendritic cells are leukocytes that are critically involved in the regulation of lymphocyte-mediated immune responses. They are professional antigen-presenting cells and are thus positioned at the beginning of virtually all immune responses, be they triggered by pathogens or, in a therapeutic intent, by physicians (vaccination). In particular, dendritic cells are the only antigen-presenting cells capable of eliciting primary immune responses, that is, responses against newly emerging antigens or threats such as for instance cancer.

Dendritic cells possess a number of cellular and molecular specializations that render them superior to other types of antigen-presenting cells. They have special uptake mechanisms for antigen; they migrate efficiently from sites of antigen uptake (e.g., skin) to sites of generation of immune responses (lymph nodes); and they are equipped with a rich set of co-stimulatory molecules that are essential for the activation of antigen-specific T lymphocytes.

These special features of dendritic cells explain why it has repeatedly been found that antigen administered to animals "loaded" onto dendritic cells led to better immune responses than antigen administered conventionally, i.e., as a peptide or protein in adjuvant. This has led Ralph M. Steinman, the original discoverer of dendritic cells, to name dendritic cells "nature's adjuvant".

The recognition of dendritic cells as prime antigen presenting cells and thus powerful inducers of protective immunity has prompted interest in harnessing them as immunogen to generate or improve immune responses in human diseases, in particular malignant tumors. Other human diseases, that are characterized by dysfunctions of the immune system (infectious diseases, autoimmune diseases, allergies, transplantation), are also amenable to therapies that make use of the immunogenic or tolerogenic properties of dendritic cells.

Based on the knowledge that tumors (melanoma, in particular) express molecules that render them immunogenic and thus vulnerable to an immune attack, and on the knowledge about the extraordinary immunostimulatory capacities of dendritic cells it has been attempted to therapeutically induce anti-cancer T cell responses by charging in vitro generated autologous dendritic cells with tumor antigens and injecting them back into tumor-bearing hosts. By generating and maturing dendritic cells ex vivo one would circumvent the non-inflammatory and even immunosuppressive milieu that presumably does not allow these T cell responses to come up in vivo, i.e., in the patient. Injected dendritic cells migrate to the lymphatic organs and present tumor antigens to the few tumor antigen-specific T cells there.

The various specialisations of dendritic cells (migratory capacity, co-stimulator expression) make it possible that tumor-specific T cells, including cytotoxic T cells will be efficiently activated and rendered functional and, as a consequence, protect or rid the organism of tumor cells. Cancer cells are killed. This concept was extensively and successfully tested in vitro and in vivo in animal models.

The initiation of clinical trials is based on and justified by broad evidence from these preclinical studies. Many mouse tumor models showed that the administration of tumor antigen-loaded autologous dendritic cells led not only to protection from subsequent challenge with tumor cells but also induced the elimination of pre-existing tumors, i.e., a clinically relevant situation. These and several other studies identified tumor antigen-specific CD8+ cytotoxic T lymphocytes as a major mechanism of tumor rejection. In addition, it was shown that also in healthy human volunteers autologous, ex vivo generated dendritic cells that had been loaded with an antigen (KLH - keyhole limpet hemocyanin) could induce potent primary T cell responses as well as immunological memory, that is, even stronger T cell responses (of the interferon-gamma-secreting Th1 type) after a second administration of dendritic cells or a second encounter with the antigen/pathogen/tumor antigen.

Thus, ample experimental evidence supports the concept that cancer can be successfully treated by using dendritic cells to induce anti-tumor immune responses. The "proof-of-principle" has been achieved. This immunological approach has at least one major advantage over many other anti-cancer strategies. The development of immunological memory will also protect from possible eventual recurrences of tumors. Another promising feature of dendritic cells has become apparent recently. Not only do they efficiently activate cytotoxic T lymphocytes but also other types of important killer cells that are involved in the body's fight against cancer cells, namely natural killer (NK) and natural killer T (NKT) cells.

Recent review articles count more than 50 published clinical trials worldwide for immunization against cancer with dendritic cells (immunotherapy; see e.g. Enk et al., J.Dtsch.Dermatol.Ges. 4 (2006), 635-644 and Schuler-Thurner et al., J. Dtsch. Dermatol. Ges. 3 (2005), 630-645). Almost all of these trials are small phase I clinical, often two-armed trials that include relatively small numbers of patients and that would initially only study toxicity and immunogenicity of the dendritic cell vaccine. Many different protocols to generate dendritic cells and to load them with tumor antigens have been applied. Likewise, dendritic cells have been administered via different routes (intradermal, intravenous, intranodal). The vaccination schedules (number of vaccinations, intervals between the vaccinations) are also different between the different studies. For these reasons it is not yet possible to statistically evaluate treatment efficacy as compared to an alternative treatment such as standard chemotherapy. However, some prominent, important and - above all - promising features have emerged from these studies:

Toxicity - side effects. Virtually all trials noted that the dendritic cell vaccines were not toxic. This includes a series of more than 450 vaccinations of the present inventors (comprising more than 50 individual patients) against melanoma, also known as "black skin cancer". Side effects were limited to some local inflammation at the cutaneous sites of injection and occasional slight fever. Quality of life was not impaired. This sharply contrasts with the known side effects of standard chemotherapy regimens.

Clinical responses. The vast majority of clinical trials has been done with patients at a late stage of their cancer disease. Therefore, not unexpectedly, clinical responses do not represent a therapeutic "breakthrough". However, encouraging clinical responses can indeed be seen in all studies. Metastases regressed. Patients showed complete remissions. DTH reactions against tumor antigens were seen in response to dendritic cells vaccinations. Survival times are markedly longer than with alternative treatment regimens. An important clinical parameter that indicates the successful induction of a T cell response in the patients' bodies is vitiligo (in the case of melanoma), that was indeed observed repeatedly in dendritic cell vaccination protocols that immunized against melanocyte differentiation antigens such as tyrosinase or MelanA/Mart-1.

Immunological responses. At present, the accurate monitoring of immunological responses is most important. Frequencies of tumor antigen-specific T cells (particularly CD8+ cytotoxic killer T cells) in the peripheral blood of patients are the most widely used and accepted parameter to monitor the immune response. In most cases these frequencies are measured by the Elispot technique, that is based on cytokine secretion of activated T cells in an antigen-specific way, and on the tetramer/pentamer technology that is based on the direct visualization of antigen-specific T cells by flow cytometry.

Using these methods tumor-antigen-specific CD8+ T cells were detected in most studies. It was shown that the induced T cells can indeed lyse tumor cells including melanoma. Importantly, when immunized with MHC class II-restricted tumor peptides, a strong induction of CD4+ helper cells of the interferon-gamma secreting type 1 was also observed. In addition, Th1 responses were also mounted in the patients against KLH, emphasizing that primary T cell responses can not only be induced by dendritic cells in healthy individuals but also in individuals with advanced disease.

Recent detailed analyses of T cell responses (mainly of T cell clones derived from responding cells) revealed a correlation between clinical responses and the numbers of tumor-specific T cell clones induced. Patients with regressing tumors showed polyclonal responses, whereas in patients that did not respond in clinical terms these authors found one clone or no clones at all. This polyclonality of T cell responses was in clear contrast to other vaccination schemes, such as the administration of tumor peptides alone or as part of a viral delivery system. This supports the rationale to employ dendritic cells for vaccination purposes rather than other approaches such as peptide vaccination.

The repeated demonstration of specific, often polyclonal, and often lytic (i.e., tumor cell killing) T cell responses against tumor antigens can thus be taken as a very strong "proof-of-principle", also in the situation of cancer-bearing patients. It justifies and necessitates the design of further studies to improve the still unsatisfactory efficacy, particularly in terms of clinical outcome.

In spite of the promising results from the first clinical trials several critical parameters still need to be improved and optimized. For example, migration of injected dendritic cells from the site of injection into the skin to the draining lymph nodes is far from being optimal. Then, the vaccination schedules need to be improved. Although immunisation in response to one single injection of antigen could be proven in normal individuals as well as in tumor patients, in most studies dendritic cells were administered several times in intervals of two or more weeks. The rationale for repeating vaccinations is that immune responses will be strengthened and immunological memory can be built up. This has indeed been shown. On the other hand, there is evidence that antigen-specific T cells may kill the immunising, antigen-bearing dendritic cells in subsequent immunisations. This is a realistic concern as such antigen-specific T cells have in fact been detected at inoculation sites of patients in the course of series of dendritic cell vaccinations. Increasing injection intervals may be a way to circumvent this potential problem. Another problem is to achieve a sufficient maturation stimulus. The current standard for maturation of dendritic cells in culture is a cytokine mix consisting of TNF-alpha, IL-1ß, IL-6, and prostaglandin E2. Dendritic cells matured by this particular cocktail induce Th1 responses in vitro and in vivo, they express CCR7, and they survive well upon withdrawal of cytokines. However, more immunogenic DCs would be beneficial. Therefore there is also a need for better maturation cocktails with additional maturation stimuli which can render dendritic cells much more immunogenic. Also the type of T cell immunity is a critical factor. While there is consensus that a strong Th1 immunity is necessary for successful cancer immunotherapy, there is also compelling evidence that Th2 immunity critically contributes to cancer eradication. Eosinophils were observed in cancer lesions and shown to mediate an anti tumor effect. Such cells are recruited to the cancer cells by mediators produced by Th2 cells. Also Th2 cells themselves were shown to mediate direct anti-cancer cell effects: they induce tumor necrosis. The clinical importance of these data may have been underestimated up to now. Finally, and perhaps the most important problem in current DC technology is the generation of large numbers of functionally potent killer cells: Monocyte-derived dendritic cells are commonly used for adoptive immunotherapy of cancer. DCs induce cytotoxic T cells, the predominantly desired effector cells in tumor therapy. Tumor regression, and thus patient benefit can only be achieved when the in vivo generation of such killer cells is successful. Large numbers of functionally potent killer cells are needed to successfully eradicate cancer cells. At present, and using currently available methods, the generation of tumor-specific killer cells is at best suboptimal. Significant improvements in the induction of killer cells will greatly enhance clinical response rates, i.e., patient benefit in terms of survival and quality of life, but it will also render dendritic cell therapy more marketable as a successful biological cancer therapy.

Based on current knowledge about DCs in tumor immunology novel approaches are urgently needed that allow the concomitant and well equilibrated generation of both types of immunity, Th1 and Th2. Furthermore, it is mandatory that high yields of such highly immunogenic cells be obtained. Neither requirement is met by any of the therapeutic modalities currently in use.

It is therefore an object of the present invention to fulfil these needs in the present field and to provide methods and means to improve DC technology. Preferably, these methods and means should enable optimal large numbers of potent killer cells and also providing Th2 immunity, including suitable maturation cocktails needed for specific immune-typing. These factors should lead to more efficient clinical use of DCs, better vaccination scheduled enabling also optimised migration results.

Therefore, the present invention provides a method for providing mature dendritic cells (DCs) which is characterised by the following steps:
- providing a monocyte preparation,
- treating the monocyte preparation with a culturing mixture comprising interleukin 4 (IL-4) to obtain a cultured preparation comprising immature DCs,
- treating the cultured preparation with a maturing mixture comprising a ligand for the toll-like receptor 3 (TLR-3 ligand) to obtain a preparation comprising mature dendritic cells (DCs).
With the present invention, a novel process for generating large numbers of mature dendritic cells from monocytes in human peripheral blood is provided. The first step is a culture of T cell depleted monocytes in medium supplemented with Interleukin-4 to produce immature dendritic cells. The second step or 'maturation' phase requires the exposure to dendritic cell maturation factors such as the combination of inflammatory mediators in the additional presence or absence of a potent inducer of Interleukin-12, namely a TLR3-ligand, such as poly I:C. These mature dendritic cells lead to the development of T cell immunity of the Th1 or the Th2 type, respectively. The present invention allows a unique combination of Interleukin-4 with a TLR3-ligand and a maturation-inducing cytokine cocktail that allows (i) the parallel and practical generation of potent Th1- as well as Th2-inducing dendritic cells, (ii) avoids the large cell losses normally encountered with the use of TLR3-ligands, such as poly I:C and other "pathogenic" nucleic acids, as the sole stimulus, and (iii) at the same time preserves the powerful IL-12-inducing capacity of the TLR3-ligand. The dendritic cells obtained by the novel process enable an improved and more efficacious dendritic cell-based immunotherapy of cancer and other diseases characterized by dysfunctions of the immune system.

The present method starts from monocytes. These can be obtained e.g. from blood by separation of lymphocytes (e.g. with magnetic beads), or by selectively binding of monocytes to surfaces and washing away lymphocytes. In general, lymphocytes should be removed to the extent practical by using standard procedures established in the field. Such a monocyte preparation may be preferably obtained from peripheral blood mononuclear cells (PBMCs). The monocyte preparation has to be present in the process according to the present invention at least in an intermediate step (i.e. without prior purification or isolation). For example, leukapheresis products can be used as a starting material to arrive at monocytes.

After the cultivating phase, monocytes have been transformed into (immature) DCs, whereafter the maturation phase follows by the addition of a maturation cocktail comprising TLR3-ligand. The resulting mature DCs then show a high capability of producing interleukin 12 (IL-12) and interleukin 23 (IL-23). The production of IL-12 and IL-23 is a decisive factor for the efficacy of dendritic cells in terms of induction of Th1-type T lymphocytes and, as a consequence, in terms of clinical responses of patients to their cancers. Mature DCs produce bioactive IL-12p70 and IL-23, albeit at relatively low levels. They do so when in contact with T lymphocytes via a molecular interaction between CD40 (on the dendritic cell side) and CD40-ligand (on the T cell side). These levels, however, may be sufficient for the induction of some level of Th1 responses in patients. Nevertheless, an augmented secretion of these cytokines will lead to increased T cell responses in patients, i.e., more and better functioning tumor killer cells. It was known that poly I:C (as a ligand for the toll-like receptor 3) can in principle induce high levels of the critical cytokine IL-12. This occurs, however, at the cost of high cell death. In other words, only very low yields of such IL-12-secreting dendritic cells can be obtained. This lowers considerably the immunogenic potential of dendritic cells and it makes long series of vaccinations practically impossible. These shortcomings precluded the use of poly I:C in dendritic cell-based therapies up to now. However, when DCs are stimulated with a combination of a maturation cocktail containing a TLR3-ligand, such as poly I:C, they produce high levels of IL-12 and IL-23 but, at the same time, remain viable. Thus, the process according to the present invention circumvents the dilemma of high IL-12 levels in only few cells. When the TLR3-ligand is omitted from the maturation step, the large numbers of resulting mature dendritic cells induce Th2 cells rather than Th1. The TLR3-ligand serves as a "switch" between the two forms of T cell immunity.

According to the present invention, a process for generating large numbers of DCs is provided, suited for immunotherapeutic purposes, from e.g. human peripheral blood in the presence of Interleukin-4 (and preferably Interleukin-3) and bringing them to full maturation by the simultaneous incubation in a maturation-inducing cytokine cocktail plus or minus the TLR3-ligand (e.g. poly I:C, the most preferred TLR3-ligand according to the present invention or other "pathogenic" nucleic acids), this leading to the development of Th1 or Th2 type T cell immunity, respectively.

This method according to the present invention is a unique combination of a Th2-promoting process for generating large numbers of dendritic cells with a potent Th-1-promoting maturation stimulus. Surprisingly, this allows to switch from Th2 to Th1 immunity in a user-defined fashion and thus, to simultaneously obtain large numbers of highly immunogenic Th1 as well as Th2-inducing dendritic cells e.g. for clinical application in immunotherapy of cancer and other diseases related to dysfunctions of the immune system.

The monocyte preparation according to the present invention is usually obtained from human blood. For therapeutic reasons, it is usually obtained from human blood of a single blood donor or of a single blood donation. Providing monocytes from other sources, such as bone marrow, is less preferred due to practical reasons.

The culturing mixture of the present invention preferably contains additional substances known in the field to be useful for DC culturing. Especially preferred are interleukin 3 (IL) and/or granulocyte macrophage colony-stimulating factor (GM-GSF) as additional culturing substances. The effects of IL-3 and GM-CSF in culturing monocytes are in principle known in the art (e.g. Ebner et al., J.Immunol. 168 (2002), 6199-6207). A standard method for generating dendritic cells from patients' peripheral blood for clinical purposes involves the use of the cytokines GM-CSF and Interleukin-4 (IL-4). Such cells induce by default a Th1 type immunity. By using IL-3, when used instead of GM-CSF, development of Th2 cells is achieved that produce less Interferon-gamma but more Interleukin-4 and Interleukin-5, typical Th2 cytokines. This may be used to activate the Th2 limb of anti-tumor immunoactivity. The use of IL-3 in addition to IL-4 in the cultivation process according to the present invention is therefore specifically preferred.

In principle, any TLR-3 ligand can be used in the method according to the present invention. However, the use of double-stranded RNA is preferred. The most preferred TLR-3 ligand according to the present invention is poly I:C. This substance is standardised and widely used in molecular biology. Poly I:C (polyinosine-polycytidylic acid) is a synthetic analog of double-stranded RNA, a molecular pattern associated with viral infection.

For maturation, any maturing mixture for DCs can be used according to the present invention (comprising the TLR3-ligand). The most preferred maturing mixture currently applied in the field additionally comprises tumor necrosis factor alpha (TNF-alpha), interleukin 1-beta (IL-1-beta), interleukin 6 (IL-6), prostaglandin E2 (PGE-2) or mixtures thereof, especially a mixture of TNF-alpha, IL-1-beta, IL-6 and PGE-2. With this mixture, currently the optimum results are achieved for maturing DCs.

Usually, the present invention also involves the loading of the DCs with specific antigens, if a specific response is to be achieved with the DCs resulting from the present invention. These antigens are normally added during or after treating with the maturing mixture, preferably during treating with the maturing mixture. If the antigens are peptidic antigens (e.g. small epitopes), addition at the end of the maturing process is preferred; more complex antigens (e.g. proteins), which need e.g. further processing, are preferably added in the course of the maturation phase.

The method according to the present invention allows the application of any antigen possible for DCs. It is therefore preferred to select the antigen from the group consisting of tumor antigens, pathogen antigens, autoimmune antigens, allergens or mixtures thereof.

The nature of the antigens is not critical, proteins can be used as well as peptidic antigens or cell lysates (e.g. tumor lysates). In a preferred embodiment of the present invention, a peptide antigen or a mixture of peptide antigens is used, preferably with a peptide length of 6 to 50 amino acid residues, more preferred of 7 to 30 amino acid residues, especially 8 to 24 amino acid residues. MHC I-restricted peptides are usually 8 to 10 amino acids long, MHC II-restricted peptides are usually 12 to 24 amino acids long. Longer versions, which comprise the minimum peptide epitopes may as well be applied.

On the other hand, it is also possible to apply the antigens (e.g. a tumor antigen) as RNA via electroporation to the cells. It is even possible to use whole cells (e.g. a dead tumor cell; "bodies") as antigens.

As stated above, the method according to the present application is specifically suitable in the field of tumor treatment and prevention. Accordingly, it is preferred to use a tumor antigen for loading the DCs according to the present invention. Preferably, the tumor antigen is selected from the group of tumor cells, tumor cell lysates, tumor peptides, or mixtures thereof, especially tumor peptides.

The present method is also well applicable in the field of prevention and treatment of pathogen-caused diseases. Therefore it is also preferred that the antigen is a pathogen antigen and the pathogen antigen is an antigen of a bacterial, fungal, eukaryotic or viral pathogen, especially an antigen selected from the group of Mycobacteria, Listeria, Salmonella, human immunodeficiency virus (HIV), Hepatitis C virus (HCV), human Papilloma virus (HPV), protozoae, especially Leishmaniae, fungal spores or mixtures of such antigens. Also with pathogens it is preferred to use a combined Th1 and Th2 attack. For example fungal pathogens are combatted by Th1 immunity against the spores and by Th2 immunity against hyphae. A mixed immune response against any challenge which needs Th1 and Th2 response can be provided with the present invention by addition or removal of the TLR3-ligand in the maturing mixture.

Preferably the present method also uses the addition of a T lymphocyte mimick to the maturing mixture, preferably CD40 ligand (CD40L). This can either be done for producing the DCs according to the present invention or - which is preferred according to the present invention - for assessment of the IL-12/IL-23 producing capabilities of the DCs produced according to the present invention.

According to a further aspect, the present invention also relates to a preparation comprising mature dendritic cells (DCs), obtainable by a method according to the present invention. The DC preparations according to the present invention are unique as they are present in a high number of viable (and therefore active) cells with Th2 inducing abilities. The maturing mixture according to the present invention allows (i) the parallel and practical generation of potent Th1- as well as Th2-inducing dendritic cells, (ii) avoids the large cell losses normally encountered with the use of TLR3-ligands, such as poly I:C, as the sole stimulus, and (iii) at the same time preserves the powerful IL-12-inducing capacity of this TLR3-ligand (especially demonstrated in the case of poly I:C). The dendritic cells according to the present invention enable improved and more efficacious dendritic cell-based immunotherapy of cancer and other diseases characterized by dysfunctions of the immune system.

A preferred preparation according to the present invention is characterised in that 1x10⁶ DCs produce supernatant levels of 50 pg or more interleukin 12 (IL-12) per ml upon stimulation with a T lymphocyte mimick, especially with CD40L, preferably 100 pg or more IL-12 per ml, especially more than 1000 pg per ml. These levels are achieved at standard conditions, i.e. at 37°C for 48 h. The IL-12 levels can be measured by any method applied in the art; preferably by an ELISA (e.g. the IL-12p70 Sandwich ELISA of R&D Systems, Minneapolis, MN, USA).

The preparation according to the present invention is preferably a preparation with DCs that induce Th1 immunity. This is characterised e.g. by an increase in IL-12 and IL-23 production. Increasing IL-12 and IL-23 production in large numbers of highly viable mature dendritic cells and having the possibility to simultaneously induce Th1 and Th2 immunity is a key factor to improve clinical responses. The present invention allows to generate dendritic cells that fulfil these three requirements.

According to a preferred embodiment of the present invention, the DC-preparation is characterised in that the preparation contains less than 10 % dead cells and preferably more than 80 %, especially more than 90 % CD83- and CCR7-expressing viable DCs. The DC-preparation is also characterised in that the absolute numbers of mature CD83- and CCR7-expressing viable DCs represent at least 30% of the cell numbers of monocytes seeded at the onset of culture.

The present invention also refers to the DC-preparation according to the present invention for medical use, i.e. a therapeutic or prophylactic preparation comprising the DCs matured according to the present invention.

A preferred embodiment of the present invention is the use of the present DCs in the tumor field. The present invention therefore relates to the use of a preparation according to the present invention for the production of a medicament for prevention or treatment of tumors, especially for the production of an autologous tumor vaccine for adoptive immunotherapy of a tumor. Clinical trials for the treatment of cancer using monocyte-derived dendritic cells have already been conducted in various clinical centres. Objective clinical responses as well as in vitro measurable T lymphocyte responses have been observed. Survival of patients was increased. As opposed to common chemotherapy regimens, these effects are not at the cost of side effects. Side effects in dendritic cell-based cancer treatment protocols are negligible and, if they rarely occur, easily tolerable and manageable. Quality of life of dendritic cell-treated patients was not impaired. Again, this contrasts sharply with current chemotherapy regimens. However, the overall clinical responses in terms of tumor regression and cure have not yet been optimal in previous set-ups. Thus, the novel DC-preparations according to the present invention represent a versatile tool for the procurement of dendritic cells that can easily be switched from Th2-promoting to Th1-promoting cells in adoptive immunotherapy of cancer. The achievement of this goal is presently hampered by the fact that dendritic cells would have to be produced in two different ways from the very beginning. This is tedious and - in view of large scale application - complicated and expensive. With the present invention this can be easily fulfilled with only one central method for the preparation of the DCs in high yields according to the present invention. The present process therefore results in high yields of IL-12 and IL-23-producing DCs that efficiently induce Th1 immunity that is critical for the generation of tumor-killing T lymphocytes. The need for high dendritic cell yields is illustrated by the fact that in currently applied vaccination schemes up to 50 million dendritic cells are administered per session (for example 4 to 10 million cells per intradermal injection). In tumor vaccination protocols used in the prior art, these dendritic cells will be injected into the skin (intradermally) using a hypodermic syringe. This is the preferred route. Alternatively, injections may be directly into the lymph nodes.

Tumor vaccination will not only be applied in the case of progressed, metastatic disease. It is also feasible in an adjuvant setting. Dendritic cell-based immunotherapy that commences immediately after surgical removal of a primary tumor is expected to prevent or delay the occurrence of metastases.

Methods are present to isolate tumor-specific T lymphocytes from tumors ("tumor infiltrating lymphocytes"). Using modern tetra- or pentamer technologies one can selectively and exclusively enrich T cells specific for one or several defined tumor antigens. Such cells can be multiplied in vitro to very large numbers and re-injected into the patient in an attempt to destroy the tumor (Gattinoni et al., Nat.Rev.Immunol. 6 (2006), 383-393). This is basically an old approach that is rendered much more specific, though, by the modern technologies. Since lymphocytes in tumors are often paralysed by factors elaborated by the cancer cells (i.e., tumor escape strategies: "the cancer strikes back") it will need autologous dendritic cells to re-activate them and make them receptive for their growth factor IL-2. The present invention allows to amplify not only the Th1 cells from the tumor but also the Th2 cells that may be there and that critically contribute to anti-tumor immunity.

The present invention also relates to the use of a DC-preparation according to the present invention for the production of a medicament for prevention or treatment of a disease caused by a pathogen, especially for a disease caused by a viral or bacterial pathogen. The present invention also bears future relevance for the treatment of infections, especially complex infections where both types of immunity may be needed. Th1 immunity is the preferred strategy of the immune system to fight against viruses and bacteria. Th2 immunity, on the other hand is the mechanism of choice against helminthic parasites, i.e., worms. Simultaneous infections by both classes of pathogens are frequent in parts of the world with poor hygienic standards. With the present invention, vaccination strategies are provided that address both limbs of the adaptive immune system, Th1 and Th2.

The DCs according to the present invention are also useable in the field of allergy treatment and prevention. Therefore, the invention relates to the use of a preparation according to the present invention for the production of a medicament for the hyposensibilisation against an allergen. Especially in cases where usual hyposensibilisation does not work, the method according to the present invention can be applied. In the case of allergies, a potent Th1 response can be made available in order to combat the Th2 response of the allergy itself.

Furthermore, the DCs obtained by the present invention may also be used for dampening or deviating dysfunctional immune responses as it is the case in autoimmunity or transplantation medicine. The preferential induction of a Th2 response, rather than the disease-causing Th1 response against autoantigens is beneficial for such patients. Therefore, the present invention also relates to the use of a preparation according to the present invention for the production of a medicament for the treatment of an autoimmune disease.

All these therapeutic or prophylactic treatments comprise the administration of an effective amount of the DCs according to the present invention to a patient in need of such treatment in order to achieve the desired effect (therapy, prevention, etc.).

In summary, as a consequence of the unique position of dendritic cells as critical regulators of immunity and tolerance, the perspectives for harnessing their potential for immunotherapy are wide and range from inducing immunity (cancer, infections) to suppressing immunity (allergy, autoimmunity, transplantation).

According to a further aspect, the present invention relates to a kit for the production of DCs comprising
- a preparation of monocytes,
- a cultivation mixture comprising IL-4 and
- a maturing mixture comprising a TLR-3 ligand.

This kit can be used to make the DCs according to the present invention. In a preferred embodiment, an additional maturing mixture may be provided in the kit which lacks the TLR-3 ligand. This would allow the parallel development of Th1 and Th2 responses based on the same monocyte preparation set-up.

Preferably, the kit according to the present invention additionally comprises a preparation of an antigen or a mixture of antigens.

According to a preferred embodiment, the kit according to the present invention comprises a maturing mixture with a TLR-3 ligand and a maturing mixture without a TLR-3 ligand. With this combination of maturing mixtures, the Th1/Th2-"switch" can easily be managed depending on the specific need. The stimulus of the TLR-3 ligand according to the present invention (especially if applied with poly I:C) is usually so strong that an almost exclusive Th1 response is enabled. Also a stepwise addition of the TLR-3 ligand to the maturing mix is possible; in this case, an isolated TLR-3 ligand preparation may additionally be present in the kit (besides a maturation mixture without the TLR-3 ligand).

A specifically preferred embodiment of the kit according to the present invention comprises the following components:
- a preparation of monocytes,
- a cultivation mixture comprising IL-4 and IL-3,
- a maturing mixture comprising poly-I:C, TNF-alpha, IL-1-beta, IL-6 and PGE-2, and
- a maturing mixture comprising TNF-alpha, IL-1-beta, IL-6 and PGE-2.

This specific embodiment is preferred i.a. due to its practicality and efficiency in clinical practice, especially for switching Th1/Th2 responses.

The present invention is further described by the following examples and the Figures, yet without being restricted thereto.
Figure 1a shows that DCs stimulated with a combination of poly I:C and the standard "maturation cocktail" (TNF-alpha, IL-1-beta, IL-6, PGE2) survive markedly better and express more CCR7 as compared to poly I:C alone;
Figure 1b shows that CD83 expression is equally well upregulated in the standard culture protocol;
Figure 2a shows that a combination of poly I:C and the standard "maturation cocktail" ( TNF-alpha , IL-1-beta, IL-6, PGE2) leads to higher cell yields as compared to poly I:C alone;
Figure 2b shows restoration of dendritic cell viability by the method according to the present invention is equally effective in the standard culture protocol;
Figure 3 shows the immunostimulatory potency of DCs procured according to the present invention;
Figure 4 shows that the combination of poly I:C and the "maturation cocktail" (TNF-alpha, IL-1-beta, IL-6, PGE2) maintains high levels of Interleukin-12 and Interleukin-23 in mature dendritic cells generated under the aegis of IL-3;
Figure 5 shows that the combination of poly I:C and the "maturation cocktail" (TNF-alpha, IL-1-beta, IL-6, PGE2) maintains high levels of IL-12 and IL-23 in mature dendritic cells, independently of their original derivation.

### EXAMPLES:

In the present example section an optimised way of performing the method according to the present invention is described. In this example, DCs according to the present invention are prepared from peripheral blood.

Step 1.: Peripheral blood mononuclear cells (PBMC) are prepared from the patients' leukapheresis products produced by the local blood bank by density centrifugation (Lymphoprep^{™} - 1.077 g/ml; Nycomed, Oslo, Norway; Ficoll Hypaque, Sigma, St. Louis, MO). Centrifugation is then continued for another 20 min at room temperature and at 460 x g (1500 rpm). Interphases are harvested and washed three times at 300 x g with ice cold PBS containing 1mM EDTA. If platelets still remain, an additional, slower wash is performed (200 x g/1000rpm for 15 min.).

Step 2.: Bulk PBMC are plated into 6-well plates at a density of 10x10⁶ per well in 3 ml of culture medium without cytokines (alternatively, monocytes can be obtained by directly enriching for CD14+ cells from PBMC by means of a magnetic sorting system (MACS^{™}, Miltenyi Biotec, Bergisch-Gladbach, Germany); purified monocytes are seeded into 6-well culture dishes at a density of 2x10⁶ monocytes per well in 3 ml of culture medium).

Step 3.: When whole PBMCs are seeded initially, the procedure continues as follows: After 2h the nonadherent cells (mostly lymphocytes) are aspirated. Warm (37°C) medium is added back to the adherent fractions and the plates are rinsed very gently in the following way: The plate is held in a tilted position. The pipette is moved along the rim of the plate in a semicircle and the medium is allowed to slowly flow over the surface of the plate. Most of the medium is aspirated back into the pipette, the plate is turned by 180°, and the procedure is repeated. The medium containing the nonadherent cells is discarded. The adherent cells are further cultured in medium containing final concentrations of 5 - 50ng/ml (equivalent to 50 - 500U/ml) of Interleukin-3 (IL-3) and 1000 - 500 U/ml IL-4. A final concentration of 10 ng/ml (equivalent to 100 U/ml) IL-3 proved optimal and was used in most assays. The described rinsing step can be ommitted when purified monocytes are used as the starting population.

Step 4.: The cultures have to be "fed" every other day, starting on d2 of culture. To this end 1 ml of culture medium is carefully aspirated from the wells. To compensate for evaporation it is replaced by 1.5 ml fresh culture medium containing 200 U/ml IL-3 and 1000 U/ml IL-4. Assuming that most of the cytokines in the cultures are used up by the time of feeding, this would yield final concentrations of 100 U/ml IL-3 and 500 U/ml IL-4.

Step 5.: On day 6 or 7 immature dendritic cells are harvested for purposes of comparison.

Step 6.: On day 6 of culture the TLR-3 ligand poly I:C (preferably at a final concentration range of 10 - 50 pg/ml; 18 pg/ml proved optimal and was used in most assays) is added to the cells in combination with the cytokine cocktail consisting of TNF-alpha (10 ng/ml), IL-1-beta (10 ng/ml), IL-6 (1.000 U/ml) and PGE2 (1 µg/ml). Parallel cultures do not contain poly I:C. Dendritic cells are cultured in this unique and hitherto undescribed maturation-inducing milieu for another 48 hours. Then, they are harvested and analysed to assess their immunostimulatory functions.

The immunostimulatory capacity of dendritic cells to induce anti-tumor immune responses is tested by the following methods and assays. In all these assays, dendritic cells matured by using the novel process modalities are compared to dendritic cells matured by conventional protocols.
- Analyses of dendritic cell viability and survival: High yields of viable dendritic cells are absolutely necessary for implementing immunotherapy. High numbers of undesired dead cells impact negatively on the critical parameters, tested below, such as expression of functionally important molecules (e.g., CD86, CCR7), T cell stimulatory capacity, IL-12 and IL-23 production. Viability is tested in the most direct and reliable way by simply counting viable cells in the hemocytometer under the microscope using trypan blue as an indicator for viability. Furthermore, it is assessed by determining the numbers of dendritic cells that undergo apoptosis, the programmed cell death. This is done by measuring by flow cytometry the expression of an apoptosis-associated serin protease (Annexin) on the cell surface of dendritic cells a well as of propidium iodide integration into DNA. Cells positive for both markers are dead.
- Analyses of the phenotype of dendritic cells: This is done on single cell suspensions using specific monoclonal antibodies and fluorescence flow cytometry ("FACS"). In particular, the expression of CCR7 is monitored. This molecule on the surface of correctly and optimally matured dendritic cells is responsible for their maximal migration from the site of injection into the skin to the draining lymph nodes where they will induce T cell responses. This is a prerequisite for successful dendritic cell-based anti-cancer immunotherapy. The chemokines CCL19 (MIP3-alpha, macrophage inflammatory protein alpha) and CCL21 (SLC, secondary lymphoid tissue chemokine) are potent chemoattractants for the migration of injected dendritic cells.
- Analyses of the T cell stimulatory capacity of dendritic cells in the classical "mixed leukocyte reaction": This stringent assay tests the very capacity of dendritic cells to induce proliferation in antigen-specific T lymphocytes, a hallmark of successful induction of an immune response including tumor killer cells. T lymphocytes in this assay are resting and naive lymphocytes, thus mimicking the in vivo situation in the lymph nodes.
- Analyses of the cytokine production of dendritic cells. This is done in response to a dendritic cell stimulus that mimicks the physiological in vivo situation when a dendritic cell enters the lymph node and meets antigen-specific (i.e., tumor-specific, in this case) T lymphocytes. A murine myeloma cell line transfected with the human CD154/CD40 ligand molecule (P3xTBA7 cells) is used for this purpose. These cells ligate the CD40 molecule on the surface of dendritic cells to induce cytokine production. IL-12p70 (the bioactive heterodimer) and IL-23 are measured by highly sensitive enzyme-linked immuno-sorbent assays (ELISA). These are the critical cytokines that are needed in substantial quantities in order to efficiently induce tumor-specific killer T cells.

Additional steps: preferably, the following further processing of DCs obtained by the process according to the present invention can be applied:
- On day 8/9 of culture the cells are harvested.
- Quality control assays are performed: Flow cytometry analyses for the expression of co-stimulator molecules CD80 and CD86, and for migration-related molecules such as CCR7; mixed leukocyte reactions for the determination of T cell stimulatory capacity; further culture in the absence of cytokines in order to test the stability of the cells.
- In the case of cancer immuntherapy, DCs are incubated with ("loaded with") tumor-specific antigens, e.g., tumor-specific peptides from MAGE, tyrosinase or gp100 proteins in the particular case of melanoma. Incubation is for 2 hours at 37°C.
- DCs are washed three times with medium.
- DCs are aspirated into 1ml syringes equipped with hypodermic needles. 4 million cells are aspirated into each syringe. Each syringe contains DCs "loaded" with different batches of tumor antigens.
- Immediately thereafter, DCs are administered to patients. They are injected into the skin (intradermally) in an area close to axillary and / or inguinal lymph nodes. DCs will enter lymph vessels of the skin and migrate there to the lymph nodes. There, they will find and expand efficiently tumor-specific T lymphocytes, including tumor killer cells.
   Typically, 10 or more such vaccinations are performed over the period of about one year. Numbers of dendritic cells produced by the present process from a patient's leukapheresis product exceed the numbers needed for one vaccination. Therefore, cells can be cryopreserved at -196°C for extended periods of time and for additional vaccinations. Appropriate aliquots are thawed immediately before a vaccination.
- The clinical course of patients is monitored by imaging techniques, such as CT, that allow the visualization of tumor metastases and permit to verify reductions in tumor mass or tumor eradication. Serological tumor markers are also monitored (such as S100 and MIA in melanoma patients). Immunological markers are followed likewise: development of killer T cells by cytotoxicity assays, ELISPOT assays, tetramer analyses in cells obtained from the blood of treated patients at defined time points during and after treatment. Finally, quality of life and patient survival are most relevant clinical parameters.

### Results:

With the present process large numbers of mature dendritic cells that produce sufficient levels of Interleukin-12 and Interleukin-23 can be obtained - a major requirement for DC-based immunotherapy. It turned out that the most potent stimulus for these two cytokines are TLR-3 ligands, especially poly I:C, according to the present invention. However, poly I:C is known as toxic to dendritic cells and therefore does not allow high cell yields. With the present method, this toxicity problem is circumvented by combining poly I:C with a cytokine cocktail consisting of four inflammatory mediators (TNF-alpha, IL-1-beta, IL-6, and PGE2) .

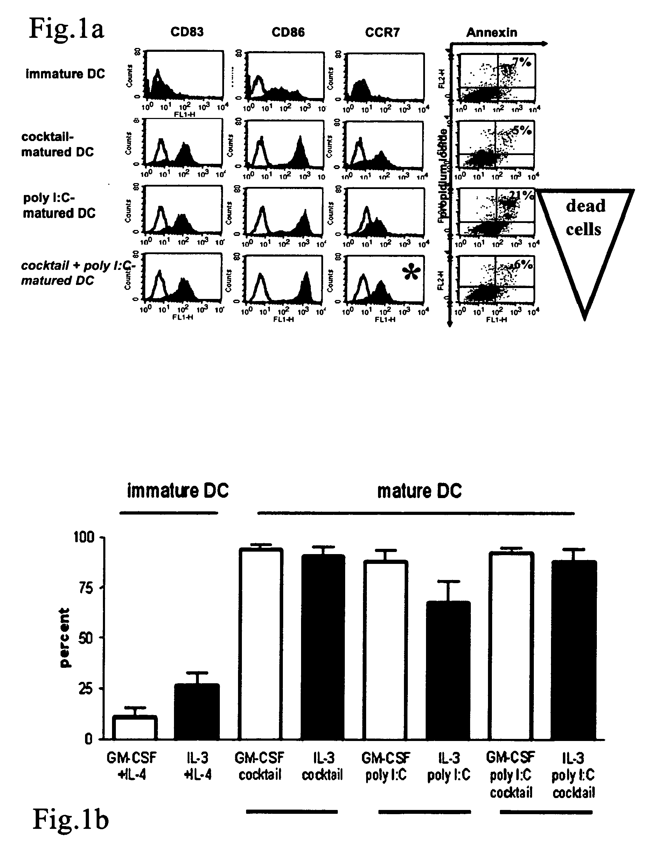

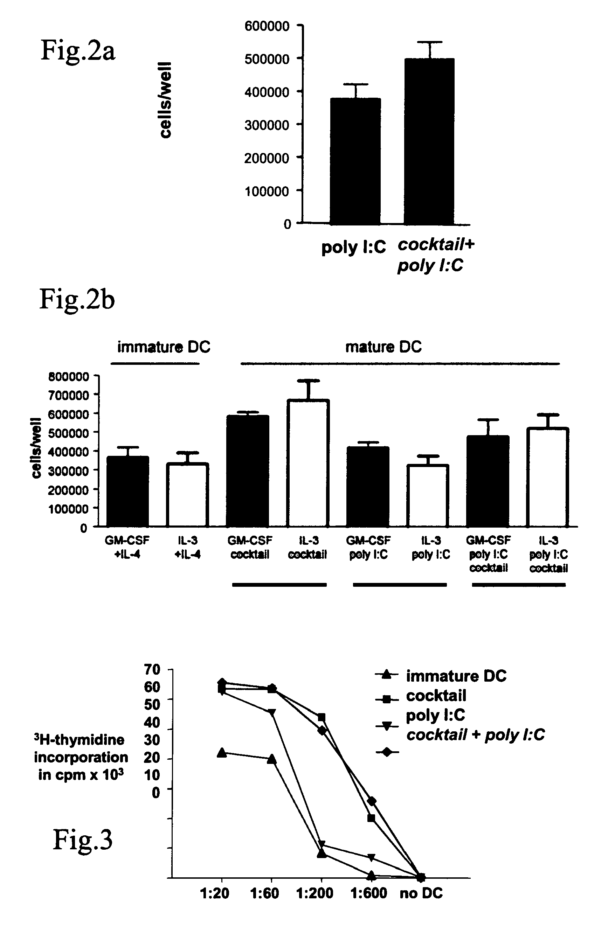

Figure 1a shows that dendritic cells brought to maturation in this manner express high levels of all the known maturation markers such as the co-stimulator molecule CD86 and the classical maturation marker CD83. Most importantly, these cells express maximal levels of CCR7, a chemokine receptor that is necessary for the migration of injected dendritic cells from the site of injection to the lymph nodes, where the anti-cancer immune reaction is initiated. Figure 1b indicates that this phenomenon is not limited to dendritic cells that have been cultured in the presence of IL-3 and IL-4, but also for the combination of GM-CSF and IL-4. Figure 2 shows that the process according to the present invention indeed fulfils the claim of rescuing high yields of dendritic cells. DCs were enumerated at the end of the 8-9 day cultures. Poly I:C as the sole maturation stimulus markedly reduced the numbers of DCs that could be retrieved from the cultures in comparison to the above-mentioned cytokine cocktail alone. The combination of the two stimuli (cocktail plus poly I:C), however, restored cell yields to almost those obtained by the cocktail alone. The classical assay for T cell stimulatory potency, the allogeneic mixed leukocyte reaction (MLR) indicated that dendritic cells stimulated with the combination of cytokine cocktail and poly I:C were equally powerful in activating T lymphocytes as those matured with cocktail alone (Figure 3).

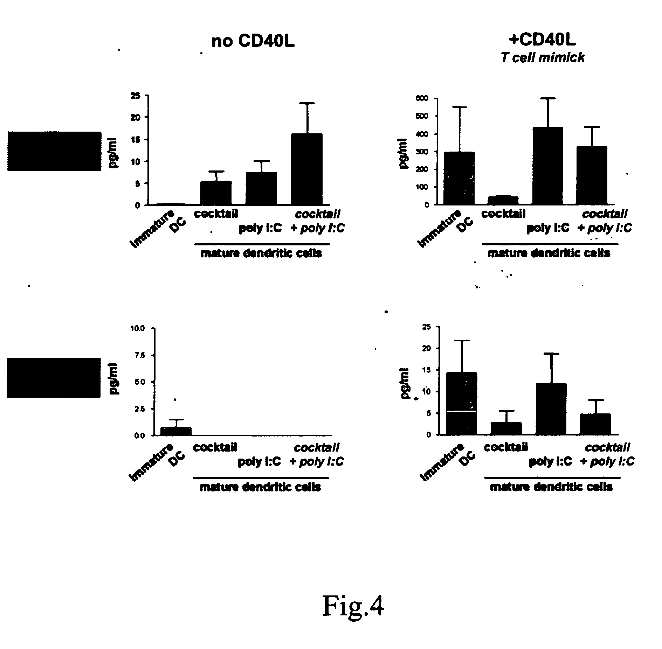

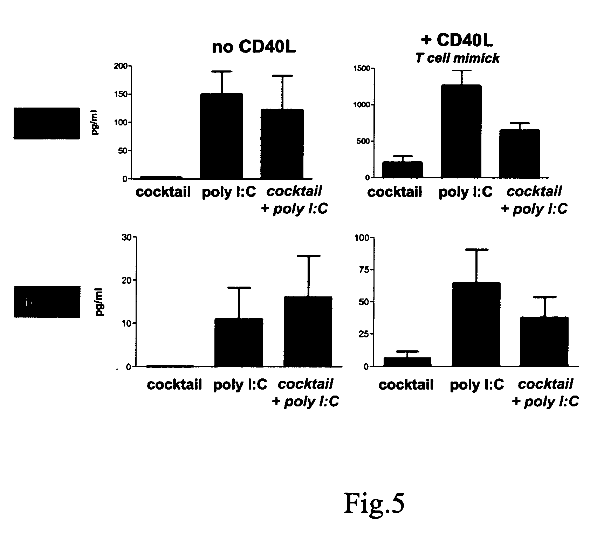

Importantly, dendritic cells matured in the combination of the cytokine cocktail and poly I:C produced high levels of Interleukin-12 and Interleukin-23 when interacting with CD40 ligand-expressing cells ("+CD40L" in Figure 4). This setting corresponds to the biologically relevant situation when the injected, tumor antigen-loaded dendritic cells enter the draining lymph node and activate tumor-specific killer T lymphocytes there. The data depicted in Figure 4 prove that those therapeutic dendritic cells would produce and secrete sufficient quantities of both critical cytokines and would thus be powerful inducers of killer cells. Figure 4 also shows that in the absence of poly I:C these mature dendritic cells do neither make IL-12 nor IL-23. This is in contrast to the standard method of generating dendritic cells in the presence of GM-CSF and IL-4; there, low, but measurable quantities of both cytokines can be detected (Figure 5). It was previously observed that dendritic cells generated in the presence of IL-3 and IL-4 induce Th2 T cells rather than Th1.

Therefore, these results show that the culture process according to the present invention allows to switch in a user-defined fashion from Th1 to Th2 immunity, both of which are important and necessary for the clinical eradication of cancer in the human body as well as for treatment of other diseases related to dysfunctions of the immune system as pointed out in the introductory part. This is depicted in the scheme below.

| | | | | | | | | |
|---|---|---|---|---|---|---|---|---|
| | | | | mature with | | mature DC | | |
| | | | → | cocktail | → | make no IL-12 | → | induction of |
| | | | | for 2 days | | and no IL-23 | | Th2 cells |
| | | culture in | ↑ | | | | | |
| monocytes | → | IL-3 + IL-4 | \| | | | | | |
| | | for 6-7 days | ↓ | | | | | |
| | | | | mature with | | mature DC | | induction of |
| | | | → | cocktail | → | make IL-I2 | → | Th1 cells |
| | | | | +poly I:C | | and IL-23 | | |
| | | | | for 2 days | | | | |

In Figure 1a it is shown that Dendritic cells stimulated with a combination of poly I:C and the standard "maturation cocktail" (TNF-alpha, IL-1-beta, IL-6, PGE2) survive markedly better and express more CCR7 as compared to poly I:C alone. The three columns to the left depict flow cytometric analyses with established maturation markers for dendritic cells. Note that the combination of cocktail plus poly I:C leads to the highest expression of CCR7 (marked by *). In the column to the right, dead cells (i.e., propidium iodide and Annexin double-positive) are indicated in the upper right quadrant of each panel. The combination of cocktail and poly I:C leads to less dead cells (indicated in % of all cells) as compared to poly I:C alone. The novel combination leads to a 3-4-fold reduction of dead cells.

In Figure 1b it is shown that CD83 expression is equally well upregulated in the standard culture protocol. This Figure shows (as in Fig. la) that the "maturation cocktail" (TNF-alpha, IL-1-beta, IL-6, PGE2) in combination with poly I:C induces expression of high levels of maturation marker CD83. Here it becomes evident that this viability-maintaining phenomenon occurs to the same extent both in dendritic cells that are generated under the aegis of GM-CSF+IL-4 (i.e., standard dendritic cells; white columns) and of IL-3+IL-4 (i.e., part of this novel process; black columns).

According to Figure 2a, a combination of poly I:C and the standard "maturation cocktail" ( TNF-alpha, IL-1-beta, IL-6, PGE2) leads to higher cell yields as compared to poly I:C alone. These cell counts reflect the flow cytometry data shown in Figure 1. Substantially fewer dendritic cells are recovered when cultured in poly I:C alone, because they die due to toxicity of poly I:C. Cell yields can be markedly increased when poly I:C is combined with the maturation cocktail.

In Figure 2b, it is shown that the restoration of dendritic cell viability by the novel combination is equally effective in the standard culture protocol. This Figure shows (as in Fig. 2a) that the "maturation cocktail" (TNF-alpha, IL-1-beta, IL-6, PGE2) prevents the drop in viability that occurs when the cells are matured in the presence of poly I:C only. Here it becomes evident that this viability-maintaining phenomenon occurs to the same extent both in dendritic cells that are generated under the aegis of GM-CSF+IL-4 (i.e., standard dendritic cells; black columns) and of IL-3+IL-4 (i.e., part of this novel process; open columns).

According to Figure 3, the immunostimulatory potency of dendritic cells procured by the process according to the present invention is depicted. In the mixed leukocyte reaction, the "gold standard" for testing the immunostimulatory potency of dendritic cells, a combination of poly I:C and the "maturation cocktail" results in high stimulatory capacity.

It should be emphasized that dendritic cell numbers in this assay refer to viable cells, i.e., this is a comparison on the basis of viable cells. If one were to test cell equivalents (as one often administers to patients in practical immunotherapy) the low viability of poly I:C matured dendritic cells would render such cell equivalents markedly lower in their immunostimulatory power as compared to the combination of poly I:C plus the cytokine cocktail.

In Figure 4 it is shown that the combination of poly I:C and the "maturation cocktail" (TNF-a, IL-1β, IL-6, PGE2) maintains high levels of Interleukin-12 and Interleukin-23 in mature dendritic cells generated under the aegis of IL-3. This is particularly true when dendritic cells are stimulated with a "T lymphocyte mimick", namely CD40 ligand ("+CD40L", right column). This is equivalent to the in vivo situation that injected, therapeutic dendritic cells would encounter when they migrate into the lymph nodes. "Cocktail" is what is used for maturation in current protocols. "Poly I:C" would yield maximal levels of IL-12 and IL-23, however, many cells die. "Cocktail + poly I:C" is the ideal modality since it combines high levels of IL-12 and IL-23 with good cell viability (see Figure 2) (The left hand side panels indicate what dendritic cells secrete in the absence of a T cell stimulus).

Figure 5 shows that the combination of poly I:C and the "maturation cocktail" (TNF-alpha, IL-1-beta, IL-6, PGE2) maintains high levels of IL-12 and IL-23 in mature dendritic cells, independently of their original derivation, i.e., irrespective of the fact how they were cultured - in the presence of IL-3+IL-4 (i.e., the process according to the present invention; depicted in Figure 4) or in GM-CSF+IL-4 (i.e., the standard method, shown in this Figure for comparison). This is particularly true when dendritic cells are stimulated with a "T lymphocyte mimick", namely CD40 ligand ("CD40L", right column). This is equivalent to the in vivo situation that injected, therapeutic dendritic cells would encounter when they migrate into the lymph nodes. "Cocktail" is what is used for maturation in current protocols. "Poly I:C" would yield maximal levels of IL-12 and IL-23, however, many cells die. "Cocktail + poly I:C" is the ideal modality since it combines high levels of IL-12 and IL-23 with good cell viability (see Figure 2) (The left hand side panels indicate what dendritic cells secrete in the absence of a T cell stimulus). It has to be noted that y-axis scales are different.

## Claims

1. : Method for providing mature dendritic cells (DCs) **characterised by** the following steps:
- providing a monocyte preparation,
- treating the monocyte preparation with a culturing mixture comprising interleukin 4 (IL-4) to obtain a cultured preparation comprising immature DCs,
- treating the cultured preparation with a maturing mixture comprising a ligand for the toll-like receptor 3 (TLR-3 ligand) to obtain a preparation comprising mature dendritic cells (DCs).

2. : Method according to claim 1 **characterised in that** the monocyte preparation was obtained from human blood, preferably from human blood of a single blood donor or of a single blood donation.

3. : Method according to claim 1 or 2, **characterised in that** the culturing mixture additionally comprises interleukin 3 (IL) and/or granulocyte macrophage colony-stimulating factor (GM-GSF), preferably IL-3.

4. : Method according to any one of claims 1 to 3, **characterised in that** the TLR-3 ligand is double-stranded RNA, preferably poly-I:C.

5. : Method according to any one of claims 1 to 4, **characterised in that** the maturing mixture additionally comprises tumor necrosis factor alpha (TNF-alpha), interleukin 1-beta (IL-1-beta), interleukin 6 (IL-6), prostaglandin E2 (PGE-2) or mixtures thereof, especially a mixture of TNF-alpha, IL-1-beta, IL-6 and PGE-2.

6. : Method according to any one of claims 1 to 5, **characterised in that** antigens are added during or after treating with the maturing mixture, preferably during treating with the maturing mixture.

7. : Method according to claim 6, **characterised in that** the antigen is selected from the group consisting of tumor antigens, pathogen antigens, autoimmune antigens, allergens or mixtures thereof.

8. : Method according to claim 6 or 7, **characterised in that** the antigen is a peptide antigen or a mixture of peptide antigens, preferably with a peptide length of 6 to 50 amino acid residues, more preferred of 7 to 30 amino acid residues, especially 8 to 24 amino acid residues.

9. : Method according to any one of claims 6 to 8, **characterised in that** the antigen is a tumor antigen selected from the group of tumor cells, tumor cell lysates, tumor peptides, or mixtures thereof, especially tumor peptides.

10. : Method according to any one of claims 6 to 8, **characterised in that** the pathogen antigen is an antigen of a bacterial, fungal, eukaryotic or viral pathogen, especially an antigen selected from the group of Mycobacteria, Listeria, Salmonella, human immunodeficiency virus (HIV), Hepatitis C virus (HCV), human Papilloma virus (HPV), protozoae, especially Leishmaniae, fungal spores or mixtures of such antigens.

11. : Method according to any one of claims 1 to 10, **characterised in that** a T lymphocyte mimick is added to the maturing mixture, preferably CD40 ligand (CD40L).

12. : Preparation comprising mature dendritic cells (DCs), obtainable by a method according to any one of claims 1 to 11.

13. : Preparation according to claim 12, **characterised in that** 1x10⁶ DCs produce supernatant levels of 50 pg or more interleukin 12 (IL-12) per ml upon stimulation with a T lymphocyte mimick, especially with CD40L, preferably 100 pg or more IL-12 per ml, especially more than 1000 pg per ml.

14. : Preparation according to claim 12 or 13, **characterised in that** the DCs induce Th1 immunity.

15. : Preparation according to any one of claims 12 to 14, **characterised in that** the preparation contains less than 10 % dead cells and preferably more than 80 %, especially more than 90 % CD83- and CCR7-expressing viable DCs.

16. : Preparation according to any one of claims 12 to 15 for medical use.

17. : Use of a preparation according to any one of claims 12 to 15 for the production of a medicament for prevention or treatment of tumors, especially for the production of an autologous tumor vaccine for adoptive immunotherapy of a tumor.

18. : Use of a preparation according to any one of claims 12 to 15 for the production of a medicament for prevention or treatment of a disease caused by a pathogen, especially for a disease caused by a viral or bacterial pathogen.

19. : Use of a preparation according to any one of claims 12 to 15 for the production of a medicament for the hyposensibilisation against an allergen.

20. : Use of a preparation according to any one of claims 12 to 15 for the production of a medicament for the treatment of an autoimmune disease.

21. : Kit for the production of DCs comprising
- a preparation of monocytes,
- a cultivation mixture comprising IL-4 and
- a maturing mixture comprising a TLR-3 ligand.

22. : A kit according to claim 21, **characterised in that** it additionally comprises a preparation of an antigen or a mixture of antigens.

23. : A kit according to claim 21 or 22, **characterised in that** it additionally comprises a maturing mixture without a TLR-3 ligand.

24. : A kit according to any one of claims 21 to 23 comprising
- a preparation of monocytes,
- a cultivation mixture comprising IL-4 and IL-3,
- a maturing mixture comprising poly-I:C, TNF-alpha, IL-1-beta, IL-6 and PGE-2, and
- a maturing mixture comprising TNF-alpha, IL-1-beta, IL-6 and PGE-2.
